(19)

Europäisches
Patentamt
European
Patent Office
Office européen
des brevets

(11)  **EP 4 282 339 A1**

(12)  **EUROPEAN PATENT APPLICATION**

(43) Date of publication:
**29.11.2023  Bulletin 2023/48**

(21) Application number: **22183472.4**

(22) Date of filing: **07.07.2022**

(51) International Patent Classification (IPC):
**A61B 8/08** *(2006.01)*        **G06T 7/00** *(2017.01)*

(52) Cooperative Patent Classification (CPC):
**A61B 8/0866; A61B 8/0883; A61B 8/5223;
G06V 10/24;** G06V 2201/03

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **25.05.2022  US 202263345805 P**

(71) Applicant: **Koninklijke Philips N.V.
5656 AG Eindhoven (NL)**

(72) Inventors:
  • **MATTUR, Shashaank Aswatha
    Eindhoven (NL)**
  • **BHOWMICK, Soumabha
    Eindhoven (NL)**
  • **RAJAGOPALAIAH, Giridhar Narasapura
    5656AG Eindhoven (NL)**

  • **AWASTHI, Manish
    5656AG Eindhoven (NL)**
  • **RAJAMANI, Kumar Thirunellai
    Eindhoven (NL)**
  • **FIRTION, Celine
    Eindhoven (NL)**
  • **VAJINEPALLI, Pallavi
    Eindhoven (NL)**
  • **MISHRA, Chandan
    Eindhoven (NL)**
  • **RIELLY, Matthew
    Eindhoven (NL)**
  • **CANFIELD, Earl
    Eindhoven (NL)**

(74) Representative: **Philips Intellectual Property &
Standards
High Tech Campus 52
5656 AG Eindhoven (NL)**

(54)  **PROCESSING SEQUENCES OF ULTRASOUND IMAGES**

(57)      According to an aspect, there is provided a method of processing a sequence of Ultrasound, US, images of an anatomical feature with periodic movements. The method comprises: i) using a first machine learning, ML, model to label detections of the anatomical feature in the images in the sequence according to view plane of the anatomical feature visible in each respective image; ii) obtaining a first cluster of consecutive images in the sequence that all correspond to a first view plane, based on the labelling; and iii) using the first cluster as a first clip of the first view plane of the anatomical feature.

300

302 → Use a first machine learning, ML, model to label detections of the anatomical feature in the images in the sequence according to view plane of the anatomical feature visible in each respective image

304 → Obtain a first cluster of consecutive images in the sequence that all correspond to a first view plane, based on the labelling

306 → Use the first cluster as a first clip of the first view plane of the anatomical feature

**Fig. 3**

EP 4 282 339 A1

**Description**

**FIELD OF THE INVENTION**

The disclosure herein relates to Ultrasound imaging. In particular, but non-exclusively, the disclosure relates to processing sequences of Ultrasound images of an anatomical feature with periodic movements.

**BACKGROUND OF THE INVENTION**

Congenital heart disease (CHD) is one of the key defects that impacts fetal health and has an effect on pregnancy outcomes. It has been observed that it affects about 1% of births in The United States per year, see paper by Hoffman JL, & Kaplan S. entitled: "The incidence of congenital heart disease. J Am Coll Cardiol. 2002;39(12):1890-1900. It has been observed that CHD can be asymptomatic, mildly symptomatic, but can become fatal after birth. According to the study by Oster ME, et al. entitled "Temporal trends in survival among infants with critical congenital heart defects" (Pediatrics. 2013 May;131(5):e1502-8. doi: 10.1542/peds.2012-3435. Epub 2013 Apr 22. PMID: 23610203; PMCID: PMC4471949), 1 in 4 babies with CHD needs to go through a surgical procedure within a year of their birth. This emphasizes the need for early detection of CHD for better therapeutic options and patient outcomes.

There are several risk factors that are associated with the presence of CHD as described in the paper by Hernandez-Andrade E, Patwardhan M, Cruz-Lemini M, Luewan S: "Early Evaluation of the Fetal Heart." (Fetal Diagn Ther 2017;42:161-173. doi: 10.1159/000477564). These include Noncardiac structural abnormalities, Previous history of CHD, Abnormal ductus venous, Increased nuchal translucency, Monochorionic twins, Aberrant right subclavian artery, Consanguinity and Assisted reproductive technologies.

Ultrasound (US) monitoring has been proven effective in early detection of anomalies in fetal hearts. However, significant manual effort is required in order to perform a fetal heart examination.

**SUMMARY OF THE INVENTION**

As noted above, significant manual processing of US images is generally needed in order to perform an US examination of a fetal heart. Extended US procedures are generally recommended for detecting the presence of any malformations in the fetal heart. Extended US examinations involve precise detection of the recommended International Society of Ultrasound in Obstetrics and Gynecology (ISUOG) and/or American Institute of Ultrasound in Medicine (AIUM) fetal heart views in order to diagnose CHD. It is an object of embodiments herein to improve the efficiency of, and reduce the manual effort currently required to perform US examinations of sequences of US images of an anatomical feature with periodic movements, such as a fetal heart.

According to a first aspect, there is provided a method of processing a sequence of Ultrasound (US) images of an anatomical feature with periodic movements. The method comprises: i) using a first machine learning, ML, model to label detections of the anatomical feature in the images in the sequence according to view plane of the anatomical feature visible in each respective image; ii) obtaining a first cluster of consecutive images in the sequence that all correspond to a first view plane, based on the labelling; and iii) using the first cluster as a first clip of the first view plane of the anatomical feature.

According to a second aspect there is an apparatus for processing a sequence of US images of an anatomical feature with periodic movements. The system comprises a memory comprising instruction data representing a set of instructions; and a processor configured to communicate with the memory and to execute the set of instructions. The set of instructions, when executed by the processor, cause the processor to: i) use a first machine learning, ML, model to label detections of the anatomical feature in the images in the sequence according to view plane of the anatomical feature visible in each respective image; ii) obtain a first cluster of consecutive images in the sequence that all correspond to a first view plane, based on the labelling; and iii) use the first cluster as a first clip of the first view plane of the anatomical feature.

According to a third aspect there is a computer program product comprising a computer readable medium, the computer readable medium having computer readable code embodied therein, the computer readable code being configured such that, on execution by a suitable computer or processor, the computer or processor is caused to perform the method of the first aspect.

In this manner, machine learning can be used in a streamlined manner to isolate clips from a full video stream of US images that correspond to particular view planes, such as the view planes required for fetal heart US examinations. This can significantly reduce the manual burden of clinical users performing US examinations.

These and other aspects will be apparent from and elucidated with reference to the embodiment(s) described hereinafter.

**BRIEF DESCRIPTION OF THE DRAWINGS**

Example embodiments will now be described, by way of example only, with reference to the following drawings, in which:

Fig. 1 is an example apparatus according to some embodiments herein;
Fig. 2 is an example ultrasound system according to some embodiments herein;
Fig. 3 is an example method according to some embodiments herein;

Fig. 4 is an example flow chart according to some embodiments herein;

Fig 5 is an example flow chart according to some embodiments herein;

Fig. 6 is an example flow chart according to some embodiments herein;

Fig. 7 shows some example correlation plots.

## DETAILED DESCRIPTION OF EMBODIMENTS

The disclosure herein relates to US examinations of anatomical features with periodic movements, such as fetal heart examinations, adult heart examinations, and examinations of the vascular structure of a patient. The methods herein may be used to extract short sequences e.g. "clips" from a sequence of US images taken as part of an US feed. In some embodiments, the clips correspond to sequences in which a constant view plane is visible, or to individual periodic cycles. Further embodiments describe methods for automatic extraction of fetal heart cycles and detection of fetal heart rate. In brief, the disclosure herein proposes AI-based workflows where ultrasound scans are bookmarked according to the fetal heart cycles and auto saved which can be used for referral or kept for documentation /later analysis. This can streamline the ultrasound workflow and make the examination faster for the sonologist.

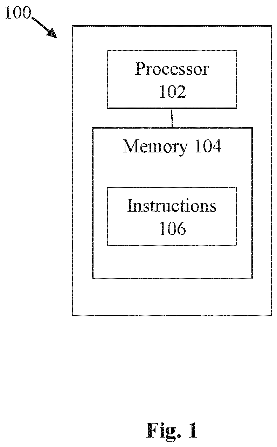

In more detail, and turning now to Fig. 1, in some embodiments there is an apparatus 100 for use in processing a sequence of US images of an anatomical feature with periodic movements, according to some embodiments herein. Generally, the apparatus may form part of a computer apparatus or system e.g. such as a laptop, desktop computer or other computing device. In some embodiments, the apparatus 100 may form part of a distributed computing arrangement or the cloud.

The apparatus comprises a memory 104 comprising instruction data representing a set of instructions and a processor 102 (e.g. processing circuitry or logic) configured to communicate with the memory and to execute the set of instructions. Generally, the set of instructions, when executed by the processor, may cause the processor to perform any of the embodiments of the method 300 as described below.

Embodiments of the apparatus 100 may be for use in processing a sequence of US images of an anatomical feature with periodic movements. More specifically, the set of instructions, when executed by the processor, cause the processor to: i) use a first machine learning, ML, model to label detections of the anatomical feature in the images in the sequence according to view plane of the anatomical feature visible in each respective image; ii) obtain a first cluster of consecutive images in the sequence that all correspond to a first view plane, based on the labelling; and iii) use the first cluster as a first clip of the first view plane of the anatomical feature.

The processor 102 can comprise one or more processors, processing units, multi-core processors or modules that are configured or programmed to control the apparatus 100 in the manner described herein. In particular implementations, the processor 102 can comprise a plurality of software and/or hardware modules that are each configured to perform, or are for performing, individual or multiple steps of the method described herein. The processor 102 can comprise one or more processors, processing units, multi-core processors and/or modules that are configured or programmed to control the apparatus 100 in the manner described herein. In some implementations, for example, the processor 102 may comprise a plurality of (for example, interoperated) processors, processing units, multi-core processors and/or modules configured for distributed processing. It will be appreciated by a person skilled in the art that such processors, processing units, multi-core processors and/or modules may be located in different locations and may perform different steps and/or different parts of a single step of the method described herein.

The memory 104 is configured to store program code that can be executed by the processor 102 to perform the method described herein. Alternatively or in addition, one or more memories 104 may be external to (i.e. separate to or remote from) the apparatus 100. For example, one or more memories 104 may be part of another device. Memory 104 can be used to store the sequence of US images, the first machine learning model, the first clip and/or any other information or data received, calculated or determined by the processor 102 of the apparatus 100 or from any interfaces, memories or devices that are external to the apparatus 100. The processor 102 may be configured to control the memory 104 to store the sequence of US images, the first machine learning model, the first clip and/or the any other information or data.

In some embodiments, the memory 104 may comprise a plurality of sub-memories, each sub-memory being capable of storing a piece of instruction data. For example, at least one sub-memory may store instruction data representing at least one instruction of the set of instructions, while at least one other sub-memory may store instruction data representing at least one other instruction of the set of instructions.

It will be appreciated that Fig. 1 only shows the components required to illustrate this aspect of the disclosure and, in a practical implementation, the apparatus 100 may comprise additional components to those shown. For example, the apparatus 100 may further comprise a display. A display may comprise, for example, a computer screen such as a Liquid Crystal Display (LCD), and/or a screen on a mobile phone or tablet. The apparatus may further comprise a user input device, such as a keyboard, mouse or other input device that enables a user to interact with the apparatus, for example, to provide input to be used in the methods described herein. The apparatus 100 may comprise a battery or other power supply for powering the apparatus 100 or means for connecting the apparatus 100 to a mains power supply.

In some embodiments, the apparatus is incorporated into an US imaging system. For example, an US imaging system may comprise the apparatus 100 and a display to display the sequence of US images and/or the first clip.

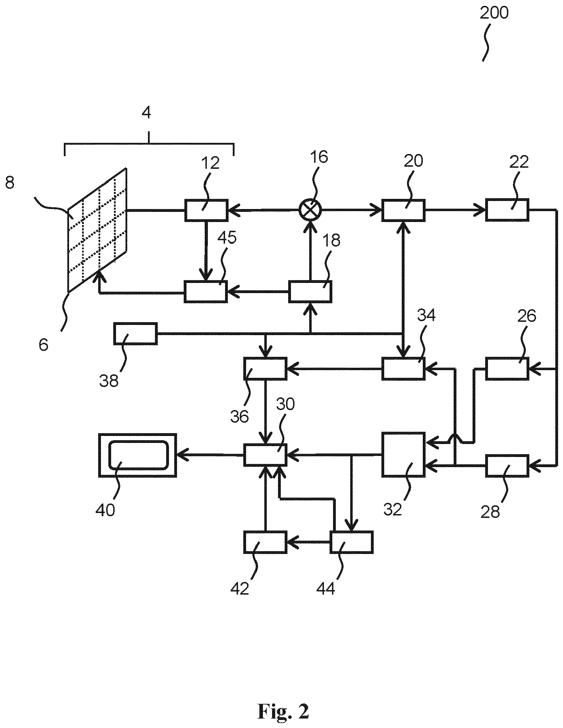

An US imaging system may further comprise other components, such as those associated with obtaining and processing US image data. An example US imaging system 200 is shown in Fig. 2. US system 200 comprises an array transducer probe 4 which has a transducer array 6 for transmitting ultrasound waves and receiving echo information. The transducer array 6 may comprise CMUT transducers; piezoelectric transducers, formed of materials such as PZT or PVDF; or any other suitable transducer technology. In this example, the transducer array 6 is a two-dimensional array of transducers 8 capable of scanning either a 2D plane or a three dimensional volume of a region of interest. In another example, the transducer array may be a 1D array.

The transducer array 6 may be coupled to a microbeamformer 12 which controls reception of signals by the transducer elements. Microbeamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

In an alternative embodiment, instead of a microbeamformer 12, the transducer array may be operated directly by a main system beamformer (not shown in Fig. 2).

The system 200 may further comprise a transmit/receive (T/R) switch 16, which the microbeamformer 12 can be coupled to and which switches the array between transmission and reception modes. The transmission of ultrasound beams from the transducer array 6 is directed by a transducer controller 18 coupled to the microbeamformer by the T/R switch 16 and a main transmission beamformer (not shown), which can receive input from the user's operation of the user interface or control panel 38. The controller 18 can include transmission circuitry arranged to drive the transducer elements of the array 6 (either directly or via a microbeamformer) during the transmission mode.

It is noted that in an alternative embodiment, instead of a microbeamformer 12, the transducer array is operated directly by a main system beamformer, a T/R switch 16 may protect the main beamformer 20 from high energy transmit signals.

In a typical line-by-line imaging sequence, the beamforming system within the probe may operate as follows. During transmission, the beamformer (which may be the microbeamformer or the main system beamformer depending upon the implementation) activates the transducer array, or a sub-aperture of the transducer array. The sub-aperture may be a one dimensional line of transducers or a two dimensional patch of transducers within the larger array. In transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal represent echoes from structures at increasing depths within the subject.

One of the functions controlled by the transducer controller 18 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 18 can be coupled to control a DC bias control 45 for the transducer array. The DC bias control 45 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the microbeamformer 12 and are then passed to a main receive beamformer 20 where the partially beamformed signals from individual patches of transducers are combined into a fully beamformed signal, referred to as radio frequency (RF) data. The beamforming performed at each stage may be carried out as described above, or may include additional functions. For example, the main beamformer 20 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 22. The signal processor 22 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and micro-bubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Fig. 2 only the receiver beamformers 12, 20 are shown, for simplicity. In the complete system, there will also be a transmission chain with a transmission micro beamformer, and a main transmission beamformer.

The function of the micro beamformer 12 is to provide an initial combination of signals in order to decrease the number of analog signal paths. This is typically performed in the analog domain.

The final beamforming is done in the main beamformer 20 and is typically after digitization.

The transmission and reception channels use the same transducer array 6 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B mode (i.e. brightness mode, or 2D imaging mode) processor 26 and a Doppler processor 28. The B mode processor 26 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 28 processes temporally distinct signals arising from tissue movement and blood flow for the detection of moving substances, such as the flow of blood cells in the image field. The Doppler processor 28 typically includes a wall filter with parameters set to pass or reject echoes returned from selected types of materials in the body.

The structural and motion signals produced by the B mode and Doppler processors are coupled to a scan converter 32 and a multi-planar reformatter 44. The scan converter 32 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 40. In the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B mode structural image with colors corresponding to motion at points in the image field, where the Doppler-estimated velocities to produce a given color. The combined B mode structural image and color Doppler image is able to depict tissue motion and blood flow within the structural image field. The multi-planar reformatter will convert echoes that are received from points in a common plane in a volumetric region of the body into an ultrasound image of that plane, as described in US Pat. 6,443,896 (Detmer). A volume renderer 42 converts the echo signals of a 3D data set into a projected 3D image as viewed from a given reference point as described in US Pat. 6,530,885 (Entrekin et al.).

The 2D or 3D images are coupled from the scan converter 32, multi-planar reformatter 44, and volume renderer 42 to an image processor 30 for further enhancement, buffering and temporary storage for display on an image display 40. The imaging processor may be adapted to remove certain imaging artifacts from the final ultrasound image, such as for example,: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 28 and tissue structure information produced by the B mode processor 26 are coupled to a quantification processor 34. The quantification processor may be used for making measurements in the images. The quantification processor may receive input from a user control panel 38.

Output data from the quantification processor is coupled to a graphics processor 36 for the reproduction of measurement graphics and values with the image on the display 40, and for audio output from the display device 40. The graphics processor 36 can also generate graphic overlays for display with the ultrasound images. These graphic overlays can contain standard identifying information such as patient name, date and time of the image, imaging parameters, and the like. For these purposes the graphics processor receives input from the user interface 38, such as patient name. The user interface is also coupled to the transmit controller 18 to control the generation of ultrasound signals from the transducer array 6 and hence the images produced by the transducer array and the ultrasound system. The transmit control function of the controller 18 is only one of the functions performed. The controller 18 also takes account of the mode of operation (given by the user) and the corresponding required transmitter configuration and bandpass configuration in the receiver analog to digital converter. The controller 18 can be a state machine with fixed states.

The user interface is also coupled to the multiplanar reformatter 44 for selection and control of the planes of multiple multi-planar reformatted (MPR) images which may be used to perform quantified measures in the image field of the MPR images.

It will be appreciated that the US image system illustrated in Fig 2 is merely an example and that an US image system may comprise different components to those described above.

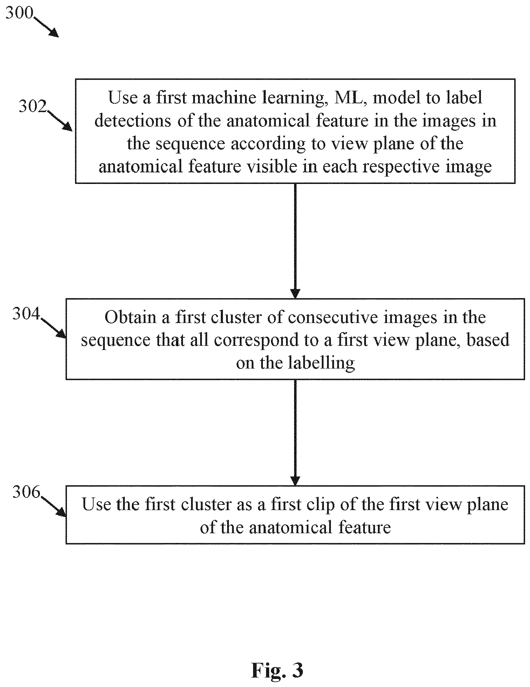

Turning now to Fig.3 which shows a computer implemented method of processing a sequence of US images of an anatomical feature with periodic movements according to some embodiments herein. The method 300 may be performed by an apparatus such as the apparatus 100 or the US system 200. In brief, in a first step 302 the method comprises, in a first step i) using 302 a first machine learning, ML, model to label detections of the anatomical feature in the images in the sequence according to view plane of the anatomical feature visible in each respective image. In a second step the method comprises ii) obtaining 304 a first cluster of consecutive images in the sequence that all correspond to a first view plane, based on the labelling; and in a third step the method comprises iii) using 306 the first cluster as a first clip of the first view plane of the anatomical feature.

The sequence of ultrasound images may be part of an ultrasound feed (e.g. ultrasound video). For example, a live ultrasound feed collected in real-time (or near-real time) as part of an ultrasound examination. Each ultrasound image in the sequence may otherwise be referred to as an ultrasound frame.

The images in the sequence of images may have been obtained using an ultrasound imaging system, such as the system illustrated in fig. 2 above. The images in the sequence of images may be two-dimensional (2D) or a three-dimensional (3D) images. The images may be comprised of image components. As used herein, image components refer to pixels in 2D or voxels in 3D. In examples where the images are 2D, then the image may be comprised of pixels; in examples where the images are 3D images, then the medical image may be comprised of voxels.

The sequence of images may have been obtained as part of an US examination of an anatomical feature with periodic movements. An anatomical feature may be all or part of an organ. Examples of anatomical features with periodic movements include, but are not limited to a fetal heart; the heart of a child, adult or animal; or part of the vascular system of a fetus, child, adult or animal. It will be appreciated however that these are merely examples and that the methods herein apply equally to other anatomical features with periodic, e.g. regularly repeating motions. Generally, the method 300 may be applied to any organ with pulsatile motion in order to measure pulsatility. For example, the lungs are another example where such pulsative movements can be observed.

In step 302, a first machine learning model is used to label detections of the anatomical feature in the images in the sequence according to view plane of the anatomical feature visible in each respective image.

In other words the first machine learning model takes an US image as input and provides as output one or more labels indicating a view plane of the anatomical feature that is visible in the image. A view plane is the 2D plane through the anatomical feature represented in the image.

As an example, in embodiments where the anatomical feature is a heart (e.g. a fetal heart, child's heart, adult heart, or animal heart), the first machine learning model may be configured to label the images according to the cardiac view planes. Example cardiac view planes include but are not limited to: 4CH, LVOT, RVOT, 3VV and 3VT view planes. Other cardiac view planes are described by the International Society of Ultrasound in Obstetrics and Gynecology (ISUOG) and/or American Institute of Ultrasound in Medicine (AIUM).

In examples where the anatomical feature is another organ, then the view planes may be standard view planes associated with that organ. More generally, view planes may be defined in any manner, for example, with respect to an angle at which the view plane bisects the organ compared to a reference plane.

The skilled person will be familiar with machine learning (ML) and machine learning processes for use in training machine learning models. ML is an approach that allows a programmer to implement a program by finding patterns in data samples. A program or model that is obtained through Machine Learning is called a Machine Learning model. The model can be used in various analysis tasks, for example classification or regression. Models are trained using training data comprising training examples, each training example comprising an example input and a corresponding "correct" ground truth output. The model is trained on the training data, using the machine learning process.

For example, the first machine learning model may have been trained using a training dataset comprising example input US images of the anatomical feature, and corresponding ground truth labels indicating the view plane visible in the respective example input image.

A machine learning process comprises a procedure that is run on the training data to create or train the machine learning model. The machine learning process thus comprises procedures and/or instructions through which training data is used to train the machine learning model. For example, if the machine learning model is a neural network, the model may be trained using processes such as back-propagation and gradient descent.

Generally the first machine learning model is configured to take an US image as input and provide an indication of a view plane visible in the image as output. In some examples, the first machine learning model may be a neural network model, such as a convolutional neural network model. Convolutional neural network models are adapted to process image data.

Other Deep Learning (DL) architectures such as U-nets, HR-nets, can also be used.

As another example, the first machine learning model may be a You Only Look Once (YOLO) network described in the paper by Redmon *et al.* (2016) entitled "*You Only Look Once: Unified, Real-Time Object Detection*". A YOLO may be trained to label US images in a sequence of US images with a label indicative of the view plane visible in each image. YOLO is adapted to process high volumes of images in image feeds such as video feeds as is therefore well suited to real-time data processing of US images with efficient performance.

The first machine learning model may be set up and trained using an opensource library and the principles set out therein, such as Scikit-Learn: Scikit-learn: Machine Learning in Python, Pedregosa etal., JMLR 12, pp. 2825-2830, 2011.

The skilled person will appreciate that these are merely examples however and that the first machine learning model could be any type of ML model that has been trained to label US images according to view plane.

In a second step the method comprises ii) obtaining 304 a first cluster of consecutive images in the sequence that all correspond to a first view plane, based on the labelling. In other words, the first cluster of images all correspond to a particular (e.g. the same) view plane and are consecutive to one another in the sequence of images.

Thus frames or images that belong to the same view plane are formed into clusters. In this regard the following term may be defined:

- Intra frame distance (L): Consider frame 'i' predicted as the LVOT view plane and the immediate next frame predicted as LVOT is i+n, in this case the intra frame distance is n.
- Therefore if the incoming frame has the intra frame distance less than L and on appending to a cluster if its size then the file is added to the cluster.
- If the incoming frame has the intra frame distance greater than L it forms a new cluster, and previous cluster algorithm is dropped.

Turning now to step 306, in the third step the method comprises iii) using 306 the first cluster as a first clip of the first view plane of the anatomical feature. The first clip is a small video/ sequence of images, all imaging the same view plane of the anatomical feature.

For example, in this step, the method 300 may comprise displaying the first clip to the user on a display associated with an ultrasound system.

The method may further comprise repeating steps i), ii) and iii) to obtain a plurality of clips of the first view plane. The plurality of clips may be displayed to the sonographer for the sonographer to be able to replay or view the different clips (or segments) of the sequence of images of the first view plane.

The method may further comprise repeating steps i), ii) and iii) to obtain a plurality of clips of different view planes of the anatomical feature. These may be displayed to the sonographer to enable the sonographer to click on and thus replay parts of an examination corresponding to a particular view plane.

The method may further comprise repeating steps i), ii) and iii) to obtain a plurality of clips of different view planes of the anatomical feature, and selecting the first clip as a preferred clip of the anatomical feature from the plurality of clips, if the first clip comprises a cluster of consecutive images for which the respective labels are more statistically significant compared to other labels in the plurality of clips. In such a manner, a "best" clip may be determined showing the portion of the sequence of images that most clearly shows the anatomical feature, taking different view planes into account. This may help the sonographer to be able to replay and e.g. make measurements in, the most appropriate view plane of the acquired US image sequence.

For example, the frequency, or period of the anatomical motions (e.g. in the case that the anatomical feature is a heart or fetal heart, the heart rate) may then be determined from the preferred clip.

Where the anatomical feature is a fetal heart, identification and recording of a best "snip"/clip (A small video/ sequence of images) for every relevant cardiac plane for the fetal heart examination deals with the problem of identification of fetal heart planes for fetal cardiac examination to capture the function of the fetal heart. The method 300 may be performed in real-time during a fetal heart examination so that a best snip corresponding to a sequence of images can be recorded in an automated manner as the user is examining the fetal heart, so that they can be used for the purpose of reporting and diagnosis.

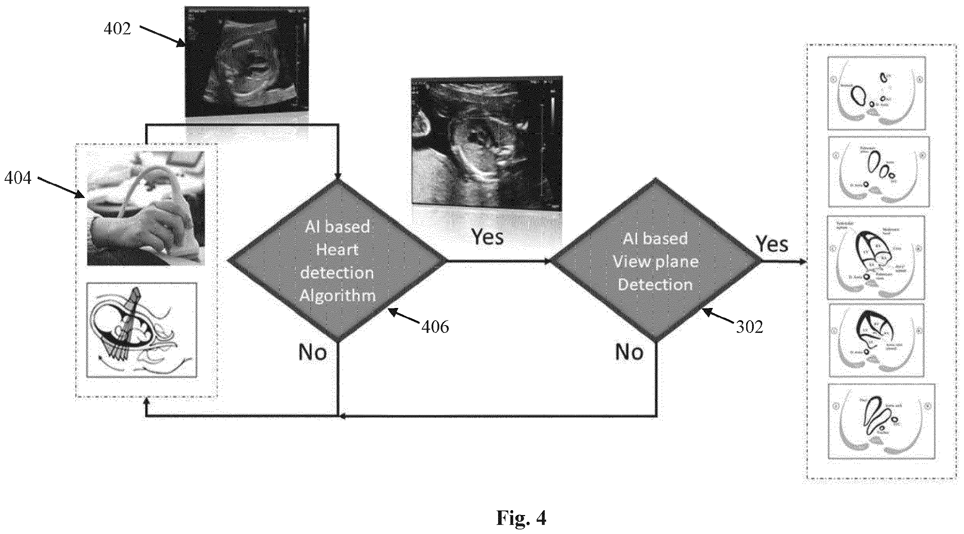

This is illustrated in Fig. 4 which shows an embodiment of the method 300 in which the anatomical feature is a fetal heart. In this embodiment, a sequence of ultrasound images 402 are acquired using an ultrasound monitor 404. At 406 a second machine learning model classifies each image as either comprising an image of the fetal heart, or not comprising an image of the fetal heart. If the heart is detected then it may crop the image to the fetal heart region. If an image comprises an image of the fetal heart then the method moves on to step 302 and a first ML model is used to label detections of the anatomical feature in the images in the sequence according to view plane of the anatomical feature visible in each respective image (in other words, the first ML model detects the fetal heart view plane which is being acquired.)

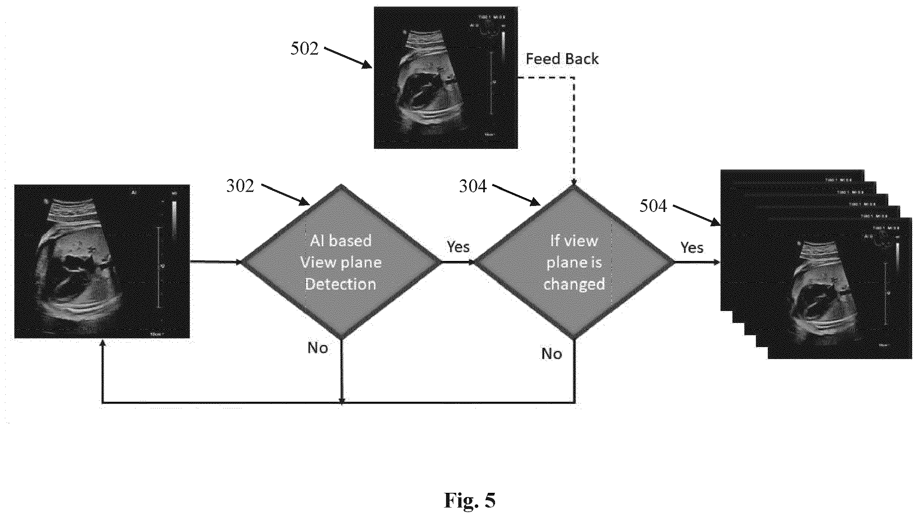

Fig 5. illustrates how that in step 304, a first cluster 504 of consecutive images in the sequence corresponding to a first view plane are obtained. In this embodiment, the start and end of a cluster is defined by images in the sequence for which the view plane changes. The clusters are then saved as a first clip of the first view plane. The detected view planes are stacked in order to form a sequence of frames.

A guidance process may be displayed on a display associated with the ultrasound scanning system to provide real time feedback 502 to the user regarding the fetal heart plane currently being imaged by the transducer probe. In this way, real-time feedback is being provided to the user.

In case of multiple clusters, and multiple heart cycles in each one of them, the one which is most statistically significant may be saved as a default. Options could also be provided to the user to choose the number of heart cycles as an output. Feedback may be provided to the sonologist to guide the sonologist to hold the probe stable so that (e.g. until) a cluster of sufficient detection quality has been obtained.

In some embodiments, if the length of the cluster is greater than a min threshold T then a heart cycle extraction and heart rate detection is performed. The heart cycle calculation is dependent on the length of the acquired sequence of frames. The minimum threshold T may be set dependent upon the frame rate of the cart and the heart rate of the fetus.

For example, if the fetal heart beats at Bm beats per minute (Bs = Bm/60) and the fps be F. Then

$$T = N \times F/Bs$$

Here the factor N is the minimum number of heart cycles needed in order to compute a statistically significant outcome. The skilled person will appreciate that the value of N may vary according to the particular circumstances, however as an example, N may be in the range 3-5.

Turning now to other embodiments, following steps 302, 304 and 306, the clips may be used to obtain synced cardiac view snips, e.g. mini-clips comprising one periodic movement of the motion, that starts and ends at a particular point in the cycle.

This can be performed in the following manner. Given a sequence of N ultrasound images, $I = \{I_1, I_2, ..., I_N\}$, from the first view plane, every image in the sequence is registered with respect to a common frame (e.g. such as the first frame in the clip) using a set of planar transformations (for example, translation, rotation, shear, affine, etc.).

Then, a minimum intensity image, $I_{min}$, is obtained (e.g. created) from the first cluster of images in the first clip. The intensity of each image component in the minimum intensity image is determined as the minimum intensity of image components in equivalent positions in each of the images in the first cluster of images. E.g. a composite image where the value of each pixel/voxel is the minimum value of equivalent pixels/voxels from the images in the first cluster of images. This is performed to obtain an image of the persistent, or stationary structures in the image.

Put another way, $I_{min}$, the Persistence-structure image, captures the non-moving structures in the images. This is calculated as the minimum of intensities at every pixel among all the images in the extracted sequence of frames. This is the base image that is used to track the position of moving objects (for e.g., valves).

The method may then comprise determining a first image, $I_{pivot}$, in the first clip that represents a turning point in the periodic motion, by comparing each image in the clip to $I_{min}$ and selecting $I_{pivot}$ as an image having either minimal or maximal intensity deviations from $I_{min}$.. The turning point, is the frame at which its difference with other frames is minimized or maximized.

The skilled person will be familiar with the term pivot frame which refers to an anchor frame in a video sequence. As used herein, a global pivot frame refers to a common starting point (or frame) with respect to the entire cineclip, and not limited to only one cycle.

The method may then comprise determining a first subset of images in the first clip corresponding to one period of the periodic movements, as images lying between the first image, and a second image representing the next consecutive turning point in the periodic motion. In other words, the first subset of images are selected as those lying between two consecutive timing points in the periodic motion (two consecutive images with maximal deviations from $I_{min}$, or two consecutive images with minimal deviations from $I_{min}$),

Once the first subset of images corresponding to a single period of the periodic motion have been obtained, a second subset may be obtained comprising a single period of the periodic motion that starts and ends at a predefined point in the cardiac cycle as follows. Firstly, an image number of a third image at a predefined phase of the periodic motion in the clip is determined. Then a relative location of the third image in the sequence compared to the first and second images is determined. A second subset of images in the first clip that start and end at the predefined phase of the motion in the clip are then selected by selecting the second subset of images relative to the first subset of images, shifted by the relative location of the third image.

In some embodiments, where the anatomical feature is a heart, the steps above may be repeated for a plurality of different predefined phases of the periodic motion. This builds up clips corresponding to one heart cycle, where the clips start and end at different points in the cardiac cycle.

In other embodiments, where the anatomical feature is a heart, the method further comprises repeating steps i), ii) and iii) for a plurality of different view planes to obtain a plurality single cycle clips that are all synchronised to a common cardiac phase (e.g. where the clips start and end at the predefined phase of cardiac motion) for display to a user. This ensures every extracted cycle begins at a common predetermined cardiac phase, for example, end systole phase.

In one embodiment, the steps described above are performed as follows on a sequence of $N$ images, $I = \{I_1, I_2, ..., I_N\}$.

The global pivot frame of the heart cycle is determined for the given heart view plane. For example, end systole in 4CH view plane, where the frame corresponding to the end systole phase is chosen as the global pivot frame, $I_{pivot}$. Among all the correlation vectors, c(t), let the correlation vector with minimum deviation be represented as $c'$.

With reference to c', frames capturing two consecutive heart cycles, $I' = \{I'_I \mid I'_{II}\} = \{I'_1, I'_2, ... I'_h \mid I'_{h+1}, I'_{h+2}, ..., \mid c \mid I,$ corresponding to an arbitrary pivot frame that is randomly seeded during the iterative process, are extracted from the original sequence of images.

From this sub-sequence of frames that capture two heart cycles, a persistent-structure image, $I_{min}$, is calculated as the minimum of intensity values among all the frames in the first cycle of the sub-sequence, $I'_I = \{I'_1, I'_2, ..., I'_h\}$.

A local reference frame, $I_{local}$, is computed by minimizing the sum of square error between $I_{min}$ and $I'_I$ which is expressed as, $I_{local} = I'_j$ such that, $argmin_j ((I_{min} - I'_j)^2), \forall j = 1, 2, ..., h.$

Then, the search space for $I_{pivot}$ is restricted between the frames $[I'_{j+(h/2)}, I'_{j+h}]$, where the pivot frames is calculated as, $I_{pivot} = I'_i$ such that, $argmax_i((I_{min} - I'_i)^2)$,

$$\forall i = j + \left(\frac{h}{2}\right), j + \left(\frac{h}{2}\right) + 1, ..., j + h.$$ That is, $I_{pivot}$ is computed by maximizing the sum of square error between $I_{min}$ and $I'_i$ in a constrained temporal range within a cycle, so that similar maxima from other frames are restricted from causing error. Also, considering two consecutive cycles preserves the continuity when $I_{local}$ occurs towards the end of the cycle. Then, in the original sequence of images, $I$, the pivot location is updated by shifting the peak locations by a step that is relative to the sequence, $I'$, and the heart cycle is extracted using the updated pivot frame in the same fashion as explained for the arbitrary pivot position. This ensures every extracted cycle begins at the pivot position of a predetermined cardiac phase, for example, end systole phase.

In this way, for a given cardiac view plane, the method 300 may be used to automatically extract cardiac cycle starting from a pivot position. For example, end systole phase for 4CH view. These may be used to display the synchronized cardiac cine clips for one or more cardiac cycles starting from end systole frame to the user. Thus the methods herein provide a manner in which to extract and display synced cardiac view snips to the sonographer.

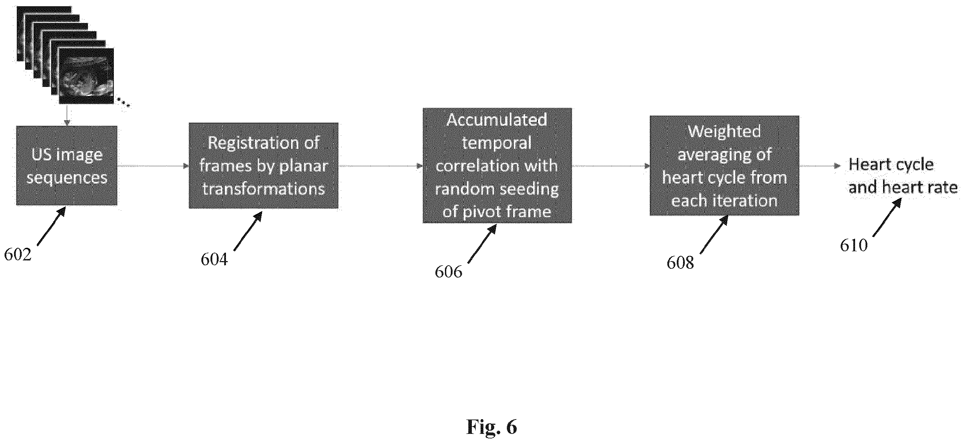

Turning now to other embodiments, Heart cycle and Heart rate detection may also be performed. The steps described in this section may be performed using clips obtained as described above with respect to the method 300. However it is noted that the steps of the method 300 are not essential to the steps that follow, and that the steps described below and with respect to Fig. 6 may equally be performed independently of the method 300. For example, the method 300 may further comprise converting each image in the first clip into a feature vector, to obtain a sequence of feature vectors.

Each feature vector may comprise an encoding of a spatial pattern in a respective image. For example, the feature vector may comprise one or more features of: a histogram of oriented gradients in the respective image; a scale invariant feature transform of the respective image; and a local binary pattern of the respective image, or any other measure that is dependent on a spatial pattern in an image.

In a further step, the method 300 may further comprise determining correlations between the feature vectors in the sequence of feature vectors; and using the correlations to determine a third subset of images from the first clip corresponding to one period of the periodic movements.

The correlations between feature vectors may be obtained by selecting a first feature vector, fp, in the sequence of feature vectors, and correlating the first feature vector fp with each of the other feature vectors in the sequence of feature vectors to obtain an N dimensional correlation vector c, wherein N is the number of images in the first clip.

Peaks in the periodic motion can then be detected by finding zero-crossings in each of the correlation vectors in a 1-dimensional Laplacian domain. In other words, the method 300 may comprise detecting peaks in the periodic signal by determining zero-crossings in a one-dimensional Laplacian domain of the correlations. An average number of images in a period of the periodic motions, may then be determined from the detected peaks. This value may be used to determine the period and/or frequency of the periodic motion, for example, if the anatomical feature is the fetal heart, it may be used to obtain fetal heart rate in an automated manner. Thus, in this manner, to detect heart cycles (as frames per cycle) in a sequence of US images, the variation of spatial pattern with respect to time can be exploited.

Turning now to an example, with an underlying assumption of having a plurality of heart cycles in a cineloop, the spatial variations in pattern of images of heart occur periodically, where each cycle consists of a systole phase and a diastole phase. Given such a sequence of $N$ ultrasound images, $I = \{I_1, I_2, ..., I_N\}$, from a static view plane, every frame in the sequence is registered with the first frame using a set of planar transformations (for example, translation, rotation, shear, affine, etc.). Although registered sequence of images is aligned by consistent structures in the images, they still have considerable local non-uniformities due to minor probe variations, noise, random smaller movements of fetus, etc. These anomalies are sufficiently strong to disturb desirable correlations between the images in the sequence.

To make the images robust to such spatial anomalies, the images are represented by local feature descriptors (for example, histogram of oriented gradients, scale invariant feature transform, speeded-up robust features, local binary pattern, etc.). Such a representation encodes the spatial pattern in the images with respective feature vectors, where each image results in a feature vector. Since all the images are registered and composed of same dimensions, all images are represented a feature vector of the same length, $D$. For $N$ images in the sequence, there are N feature vectors of $D$-dimensions, which are represented by, $\{f_1, f_2, f_N\}$ where $f_1$ to $f_N$ are the feature descriptors or region descriptors. Then, these feature vectors are correlated and accumulated temporally to detect occurrences of repeating patterns.

The correlation of feature vectors is performed in an accumulative way, which is iterated T times. Cumulated correlation is a process where several coherent correlation vectors are averaged (or summed) to form a single correlation vector that is normalized up to a scale of consideration. This averaged correlation vector is representative of correlation pattern from multiple temporal scales, realized by accumulation by averaging. Temporal scales are determined by the number of frames that are stacked to form the combined feature vector. When only one frame is considered, it is the finest scale (acquisition resolution), and the scale grows coarser by using more number of frames.

In the $t^{th}$ iteration ($t = 1, 2, ..., T$), a seed position is randomly chosen within the sequence as a pivot frame, $I_p$ (where, $p \in \{1, 2, ..., T\text{-}K\}$). Then, the D-dimensional feature vector, $f_p$, of the pivot frame is correlated with each of the D-dimensional feature vectors of all the other frames in the sequence, which results in a N-dimensional correlation vector at $t^{th}$ iteration, $c^{(t)}_1$. Each element of $c^{(t)}_1$ corresponds to a correlation score of feature vectors of the pivot frame, $f_p$, and any other frame in the sequence, $f_i$.

Put another way, an arbitrary frame may be chosen as the "pivot frame". And a correlation between the feature vector of the pivot frame and each of the other feature vectors is then determined. Each correlation is a number (score), so this produces a vector of correlation values between the pivot frame and each other frame in the sequence.

Then, a second correlation vector, $c^{(t)}_2$, is computed by correlating a $D \times 2$-dimensional feature matrix, which is formed as a 2-tuple feature vector by stacking the feature vectors, $f_p$ and $f_{p+1}$, from the pivot frame and its next frame. By stacking, we compose a combined feature vector by appending individual feature vectors of pivot frame and its next consecutive frame. During correlation, similarly combined feature vectors from every two consecutive frames are considered to determine the correlation value (score). Here, c(t)2 represents two consecutive frames under consideration. Similarly, in the next step, c(t)k represents considering k consecutive frames to form the combined feature vector. This $D \times 2$-dimensional feature matrix is correlated with all other 2-tuple feature vector combinations that are formed by feature vectors of every two consecutive frames.

More generally, $c^{(t)}_k$ represents the correlation vector of k tuple of feature vectors in $t^{th}$ iteration. Similarly, this process is repeated K times by incrementing the tuple combination of feature vectors each time by a unit count. The $K^{th}$ correlation vector, $c^{(t)}_K$, is formed by considering K-tuple feature vectors, $f_p, f_{p+1}, ... , f_{p+K}$, as a $D \times K$-dimensional feature matrix, which is correlated with all other K-tuple feature vector combinations that are formed by feature vectors of every K consecutive frames in the sequence. The mean of the correlation vectors from all K tuple combinations in the $t^{th}$ iteration is computed as the final correlation vector of $t^{th}$ iteration, $c^{(t)}$, which is given by,

$$c^{(t)} = \frac{1}{K} \sum_{k=1}^{K} c^{(t)}_k .$$

Repeating this process over T iterations results in T correlation vectors, $c^{(1)}, c^{(2)}, ..., c^{(T)}$, which correspond to different randomly chosen pivot frames. The iterative computations with random seeding of pivot frames make the system robust to temporal anomalies that may arise from discontinuities in temporal structures due to several discrepancies, for example missing frames, damaged frames, motion blur, poor resolution, etc. In each of the T correlation vectors, the starting location of the tuple of frames that are similar to the tuple formed by pivot frame and its subsequent frames have comparatively higher values, which result in respective peaks of values periodically. The peaks are detected by finding zero-crossings in each of the correlation vectors in 1-dimensional Laplacian domain. The difference between the peak locations essentially determine the number of frames between the onset of two consecutive heart cycles, which is the number of frames in each heart cycle (or simply, heart cycle). Since there are multiple heart cycles in each sequence of images, in $t^{th}$ iteration, the mean of the difference between every consecutive peak locations gives the average number of frames in a heart cycle, $h^{(t)}$, and the standard deviation of the difference between every consecutive peak locations represents the quality of the computed heart cycle, $s^{(t)}$. Ideally, $s^{(t)}$ is desired to be zero. However, due to practical variability, a value of $s^{(t)} > 0$ is often observed. To have a robust estimation of heart cycle, a weighted average of the values of heart cycles that are obtained at each iteration is computed. The weights are chosen by functions that are inversely proportional to the standard deviation of the heart cycle measurements in each of the iterations, which give a statistically consistent estimate of the heart cycles in terms of frames per cycle. The estimated heart cycle is computed as, $h = \sum_{t=1}^{T} w^{(t)} \cdot h^{(t)}$ , where,

$w^{(t)} \propto \frac{1}{s^{(t)}+1}$ and $(\cdot)$ represents product of terms. The heart rate in beats per minute may then be computed as,

$\frac{60 \cdot F_{acq}}{h}$ bpm, where, $F_{acq}$ is the frequency of ultrasound image acquisition. Also, a statistical confidence score is

computed as, $\chi_c = \max \left( \left(1 - \frac{\bar{s}}{h}\right), 0 \right)$, where, $\chi_c$ is the confidence score, and $\bar{s}$ is the average of $s^{(t)}$, and the value of $\chi_c$ ranges between [0,1]. Ideally, $\chi_c = 1$, for the case of normal heart rate. Lower values of $\chi_c$, based on an empirically set threshold, $\chi_c < \tau_s$, denote abnormal heart rate. The score may also indicate anomaly in the acquisition process, which influences the periodicity of heart cycles.

The steps above are summarized in Fig. 6 which shows steps of US image acquisition 602, Image registration 604, and the step of accumulating temporal correlations between images in the sequence 606, as described above. Fig. 6 further shows steps of taking a weighted average 608 of the heart cycle obtained in each iteration (e.g. each cluster) and determining the heart cycle and the heart rate therefrom in step 610.

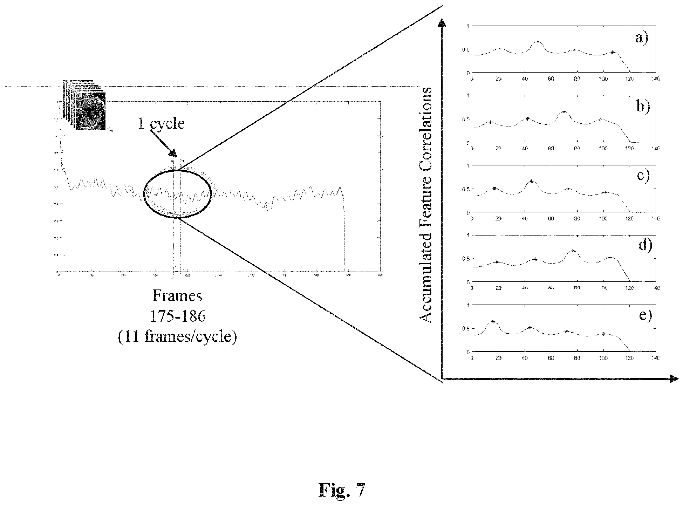

Fig. 7 shows a schematic plot of correlation vectors and a diagrammatic description of heart cycle and correlation peaks. This is provided as an example, for reference purposes. The left part of Fig. 7 represents a plot of correlation vectors from one iteration. Diagrammatic representation of one heart cycle and frames in it are also shown. The right diagram shows the plot of correlation vector from five iterations a)-e), which are used to estimate the final heart cycle in frames per cycle by their weighted averaging.

In this way, a Heart/ Cardiac Cycle can be extracted from a clip/snip: Automated extraction of one or more heart/ cardiac cycles from the detected snip corresponding to a view plane including identification of the end diastolic frame in the cardiac cycle for all the cardiac view planes (e.g. such as 4CH, LVOT, RVOT, 3VV and/or 3VT).

As noted above, the heart cycles extracted from a cineloop/live scanning can then be used to compute the fetal heart rate of the fetus.

Although the examples above primarily describe the application of the method 300 to fetal heart examinations, the skilled person will appreciate that the method can equally be used to solve any periodicity detection problem of ultrasound.

The embodiments described above provide, amongst others, the following: statistically robust determination of fetal heart rate using only ultrasound image sequences. Extraction of best / required number of clustered ultrasound frames in a sequence of images that correspond to individual heart cycles of the fetus. Determination of abnormality in heart rate, which violates the periodicity of temporal variation in spatial pattern of fetal heart ultrasound images. Extraction of bookmarkable cine-loops from a continuous sequence of fetal heart ultrasound images. Extraction of a plurality of cine-loops that are pivoted at a common cardiac phase, for example, end-systole phase. Assistance: Provides cues of frame indices that appear to have non-uniform heart cycles, which may either be the result of probe / fetus movement, or abnormality in fetal heart.

In another embodiment, there is provided a computer program product comprising a computer readable medium, the computer readable medium having computer readable code embodied therein, the computer readable code being configured such that, on execution by a suitable computer or processor, the computer or processor is caused to perform the method or methods described herein.

Thus, it will be appreciated that the disclosure also applies to computer programs, particularly computer programs on or in a carrier, adapted to put embodiments into practice. The program may be in the form of a source code, an object code, a code intermediate source and an object code such as in a partially compiled form, or in any other form suitable for use in the implementation of the method according to the embodiments described herein.

It will also be appreciated that such a program may have many different architectural designs. For example, a program code implementing the functionality of the method or system may be sub-divided into one or more sub-routines. Many different ways of distributing the functionality among these sub-routines will be apparent to the skilled person. The sub-routines may be stored together in one executable file to form a self-contained program. Such an executable file may comprise computerexecutable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solidstate medium supplied together with or as part of other hardware, but may also be distributed in other forms, such as via the Internet or other wired or wireless telecommunication systems. Any reference signs in the claims should not be construed as limiting the scope.

**Claims**

1. A computer implemented method of processing a sequence of Ultrasound, US, images of an anatomical feature with periodic movements, the method comprising:

using (302) a first machine learning, ML, model to label detections of the anatomical feature in the images in the sequence according to view plane of the anatomical feature visible in each respective image;
obtaining (304) a first cluster of consecutive images in the sequence that all correspond to a first view plane, based on the labelling; and
using (306) the first cluster as a first clip of the first view plane of the anatomical feature.

2. A method as in claim 1 further comprising:

repeating steps i), ii) and iii) to obtain a plurality of clips of different view planes of the anatomical feature; and
selecting the first clip as a preferred clip of the anatomical feature from the plurality of clips, if the first clip comprises a cluster of consecutive images for which the respective labels are more statistically significant compared to other labels in the plurality of clips.

3. A method as in claim 2 further comprising:
determining a frequency of the periodic motions from the preferred clip.

4. A method as in claim 1, 2 or 3 further comprising:
determining a minimum intensity image, $I_{min}$, from the first cluster of images in the first clip, wherein the intensity of each image component in the minimum intensity image is determined as the minimum intensity of image components in equivalent positions in each of the images in the first cluster of images.

5. A method as in claim 4 further comprising:
determining a first image, $I_{pivot}$, in the first clip that represents a turning point in the periodic motion, by comparing each image in the clip to $I_{min}$ and selecting $I_{pivot}$ as an image having either minimal or maximal intensity deviations from $I_{min}$.

6. A method as in claim 5 further comprising:
determining a first subset of images in the first clip corresponding to one period of the periodic movements, as images lying between the first image, and a second image representing the next consecutive turning point in the periodic motion.

7. A method as in claim 6 comprising:

determining an image number of a third image at a predefined phase of the periodic motion in the clip; and
determining a relative location of the third image in the sequence compared to the first and second images; and
determining a second subset of images in the first clip that start and end at the predefined phase of the motion in the clip by selecting the second subset of images relative to the first subset of images, shifted by the relative location of the third image.

8. A method as in claim 7 wherein the anatomical feature is a heart and the method further comprises:

repeating steps i), ii) and iii) for a plurality of different predefined phases of the periodic motion; and/or
repeating steps i), ii) and iii) for a plurality of different view planes to obtain a plurality single cycle clips that are all synchronised to a common cardiac phase for display to a user.

9. A method as in any one of the preceding claims further comprising:

converting each image in the first clip into a feature vector, to obtain a sequence of feature vectors; determining correlations between the feature vectors in the sequence of feature vectors; and
using the correlations to determine a third subset of images from the first clip corresponding to one period of the periodic movements.

10. A method as in claim 9 wherein the feature vector comprises:
an encoding of a spatial pattern in a respective image; and/or
one or more features of:

a histogram of oriented gradients in the respective image;
a scale invariant feature transform of the respective image; and
a local binary pattern of the respective image.

11. A method as in claim 9 or 10 wherein step v) comprises:

selecting a first feature vector, $f_p$, in the sequence of feature vectors;
correlating the first feature vector $f_p$ with each of the other feature vectors in the sequence of feature vectors to obtain an N dimensional correlation vector c, wherein N is the number of images in the first clip.

12. A method as in any one of claims 9 to 11 wherein in step vi) the method comprises:
detecting peaks in the periodic signal by determining zero-crossings in a one-dimensional Laplacian domain of the correlations.

13. A method as in claim 12 further comprising: determining an average number of images in a period of the periodic motions, from the detected peaks.

14. An apparatus for processing a sequence of Ultrasound, US, images of an anatomical feature with periodic movements, the system comprising:

a memory comprising instruction data representing a set of instructions; and a processor configured to communicate with the memory and to execute the set of instructions, wherein the set of instructions, when executed by the processor, cause the processor to:

use a first machine learning, ML, model to label detections of the anatomical feature in the images in the sequence according to view plane of the anatomical feature visible in each respective image; obtain a first cluster of consecutive images in the sequence that all correspond to a first view plane, based on the labelling; and use the first cluster as a first clip of the first view plane of the anatomical feature.

15. A computer program product comprising a computer readable medium, the computer readable medium having computer readable code embodied therein, the computer readable code being configured such that, on execution by a suitable computer or processor, the computer or processor is caused to perform the method as claimed in any one of claims 1 to 13.

100

Processor
102

Memory 104

Instructions
106

**Fig. 1**

**Fig. 2**

300

302 ┌─────────────────────────────────────────────┐
│ Use a first machine learning, ML, model to label │
│ detections of the anatomical feature in the images in │
│ the sequence according to view plane of the │
│ anatomical feature visible in each respective image │
└─────────────────────────────────────────────┘

304 ┌─────────────────────────────────────────────┐
│ Obtain a first cluster of consecutive images in the │
│ sequence that all correspond to a first view plane, based │
│ on the labelling │
└─────────────────────────────────────────────┘

306 ┌─────────────────────────────────────────────┐
│ Use the first cluster as a first clip of the first view plane │
│ of the anatomical feature │
└─────────────────────────────────────────────┘

**Fig. 3**

**Fig. 4**

**Fig. 5**

**Fig. 6**

**Fig. 7**

Europäisches
Patentamt

European
Patent Office

Office européen
des brevets

# EUROPEAN SEARCH REPORT

Application Number

EP 22 18 3472

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/178404 A1 (UNIV CALIFORNIA [US]) 19 September 2019 (2019-09-19) | 1,4-9, 14,15 | INV. A61B8/08 G06T7/00 |
| Y | * paragraph [0026] * <br> * paragraph [0040] * <br> * paragraph [0043] * <br> * paragraph [0047] * <br> * paragraph [0107] * <br> * figures 2A, 2B * | 10-13 | |
| Y | US 2006/064017 A1 (KRISHNAN SRIRAM [US] ET AL) 23 March 2006 (2006-03-23) <br> * paragraph [0066] * <br> * paragraph [0046] - paragraph [0048] * | 10-13 | |

TECHNICAL FIELDS
SEARCHED      (IPC)

A61B
G06T

The present search report has been drawn up for all claims

| Place of search | Date of completion of the search | Examiner |
|---|---|---|
| Munich | 19 December 2022 | Ordavo, Ivan |

EPO FORM 1503 03.82 (P04C01)

## ANNEX TO THE EUROPEAN SEARCH REPORT
## ON EUROPEAN PATENT APPLICATION NO.

EP 22 18 3472

This annex lists the patent family members relating to the patent documents cited in the above-mentioned European search report.
The members are as contained in the European Patent Office EDP file on
The European Patent Office is in no way liable for these particulars which are merely given for the purpose of information.

19-12-2022

| Patent document cited in search report | Publication date | Patent family member(s) | Publication date |
|---|---|---|---|
| WO 2019178404 A1 | 19-09-2019 | US 2021000449 A1<br>WO 2019178404 A1 | 07-01-2021<br>19-09-2019 |
| US 2006064017 A1 | 23-03-2006 | US 2006064017 A1<br>WO 2006034366 A1 | 23-03-2006<br>30-03-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5997479 A, Savord
- US 6013032 A, Savord
- US 6623432 B, Powers
- US 6283919 B, Roundhill
- US 6458083 B, Jago
- US 6443896 B, Detmer
- US 6530885 B, Entrekin

### Non-patent literature cited in the description

- **HOFFMAN JL ; KAPLAN S.** The incidence of congenital heart disease. *J Am Coll Cardiol,* 2002, vol. 39 (12), 1890-1900
- **OSTER ME et al.** Temporal trends in survival among infants with critical congenital heart defects. *Pediatrics,* 22 April 2013, vol. 131 (5), e1502-8
- **HERNANDEZ-ANDRADE E ; PATWARDHAN M ; CRUZ-LEMINI M ; LUEWAN S.** Early Evaluation of the Fetal Heart. *Fetal Diagn Ther,* 2017, vol. 42, 161-173
- **PEDREGOSA et al.** Scikit-Learn: Scikit-learn: Machine Learning in Python. *JMLR,* 2011, vol. 12, 2825-2830